# FASCICULE DE BREVET EUROPEEN

(11) **EP 1 447 076 B1**
(45) Date de publication et mention de la délivrance du brevet: **16.08.2006**
(21) Numéro de dépôt: 04290257.7
(22) Date de dépôt: 02.02.2004
(51) Int. Cl.: A61K 8/19, A61Q 5/12

(54) **Utilisation de solutions de fullerènes pour le conditionnement des cheveux**
Verwendung von Fullerenlösungen zur Konditionierung von Haaren
Use of fullerene solutions for conditioning hair

(30) Priorité: 12.02.2003 FR 0301673
(43) Date de publication de la demande: 18.08.2004
(73) Titulaire: L'ORÉAL, 75008 Paris (FR)
(72) Inventeur: Giroud, Franck, 92110 Clichy (FR)
(74) Mandataire: Casalonga, Axel

(56) Documents cités:
- EP-A- 0 599 687
- DE-A- 4 421 207
- DATABASE WPI Section Ch, Week 199802 Derwent Publications Ltd., London, GB; Class D21, AN 1998-014649 XP002256981 & JP 09 278625 A (SHISEIDO CO LTD) 28 octobre 1997 (1997-10-28)

## Description

L'invention concerne l'utilisation de fullerènes à l'état dissous dans un ou plusieurs solvants organiques, pour le conditionnement ou l'augmentation de la brillance des cheveux, ainsi que des compositions cosmétiques contenant des fullerènes à l'état dissous dans un ou plusieurs solvants organiques.

Il est connu d'utiliser des composés fluides à basse tension superficielle tels que les huiles de silicones non-volatiles pour faciliter le démêlage des cheveux à l'état sec et humide. Ces composés, qui agissent comme des agents lubrifiants des cheveux, permettent d'obtenir un excellent effet de conditionnement de la fibre kératinique mais leur utilisation s'accompagne de certains défauts cosmétiques tels qu'un toucher gras ou chargé ou encore un transfert de matière sur des matériaux venant en contact avec les cheveux.

On ne connaît actuellement pas d'agent lubrifiant permettant l'obtention d'un niveau de conditionnement des cheveux équivalent à celui des silicones mais ne présentant pas les inconvénients de celles-ci.

La demanderesse a découvert de manière fortuite qu'il était possible d'obtenir un excellent effet de conditionnement et de brillance des cheveux en utilisant, en tant que principe actif, une forme particulière du carbone connue sous le nom de "fullerènes". L'effet de conditionnement et d'augmentation de la brillance recherché n'est toutefois obtenu qu'à la condition que les molécules de fullerènes soient appliquées sur les cheveux à l'état dissous.

L'invention a par conséquent pour objet l'utilisation de fullerènes non-modifiés par greffage de fonctions chimiques sur le squelette carboné, à l'état dissous dans un ou plusieurs solvants organiques comportant au moins un noyau aromatique, pour le conditionnement et/ou l'augmentation de la brillance des cheveux.

L'invention a également pour objet un procédé de traitement des cheveux, comprenant l'application et l'étalement sur les cheveux, d'une solution de fullerènes non-modifiés par greffage de fonctions chimiques sur le squelette carboné, dans un ou plusieurs solvants organiques comportant au moins un noyau aromatique, éventuellement le rinçage à l'eau des cheveux ainsi traités, puis le séchage des cheveux.

L'invention a encore pour objet des compositions cosmétiques, destinées au traitement des cheveux, contenant des fullerènes non-modifiés par greffage de fonctions chimiques sur le squelette carboné, à l'état dissous dans un ou plusieurs solvants organiques choisis parmi les esters d'acides mono- ou dicarboxyliques comportant au moins un noyau aromatique.

Les fullerènes sont, à côté du graphite et du diamant, une troisième forme du carbone, découverte dans les années 80. Il s'agit de molécules constituées d'un nombre déterminé d'atomes de carbone et ayant la forme de cages fermées. Ces cages sont formées de 12 pentagones et d'un nombre variable d'hexagones qui dépend du nombre d'atomes de carbone de la molécule. La forme de fullerènes la plus stable est une molécule constituée de 60 atomes de carbone (C₆₀), mais il existe d'autres formes stables comportant de 20 à 600 atomes de carbone. On pourra trouver une description détaillée de ces molécules dans les articles et ouvrages suivants :
E. R. Berstein, Physical and Theoretical Chemistry, volume 68, Elsevier Science, New York, 1990, pages 1 - 68,
R.F. Curl et al. "Fullerenes", Scientific American, octobre 1991, pages 32 - 41,
F. Diederich et al. "The Higher Fullerenes : Isolation and Characterization", Science, volume 252, avril 1991, pages 548 - 551,
R. E. Smalley, "Great Balls of Carbon : The Story of Buckminsterfullerene", The Sciences, volume 31, n° 2, mars-avril 1991, pages 22 - 26, et
D. Koruga et al. "Fullerenes C60 - History, Physics, Nanobiology, Nanotechnology", Elsevier Science Publishers B. V., Pays-Bas, 1993.

Les fullerènes au sens de la présente invention englobent uniquement les formes non-modifiées par greffage de fonctions chimiques sur le squelette carboné.

Parmi les fullerènes existants, on utilise de préférence dans les compositions de la présente invention les fullerènes constitués de 60, 70 ou 84 atomes de carbone, et en particulier la forme la plus stable constituée de 60 atomes de carbone (C₆₀).

L'utilisation de ces molécules en tant que charges ou pigments, c'est-à-dire sous forme insoluble, dans des compositions de maquillage des yeux est décrite dans la demande de brevet européen EP 0 599 687.

L'application des fullerènes sous forme insoluble ne permet toutefois pas l'obtention de l'effet de conditionnement recherché.

Le brevet JP 9278625 décrit des compositions cosmétiques photoprotectrices contenant des fullerènes "solubilisés" dans un composant huileux (agent "solubilisant") de formule R₁-O-(C=O)-R₃-(C=O)-O-R₂ où R₁ à R₃ représentent chacun indépendamment un résidu alkyle ou alkylène en C₁₋₁₈.

Ce document ne divulgue à aucun moment l'utilisation de ces compositions pour le traitement des cheveux, en particulier pour le conditionnement ou l'augmentation de la brillance des cheveux.

Par ailleurs, la demanderesse, lors d'essais ayant pour but d'évaluer le pouvoir conditionneur des compositions divulguées dans ce document, a constaté que les fullerènes C₆₀ introduits dans un des diesters aliphatiques exemplifiés dans ce document, ne formaient en fait pas de mélanges présentant la couleur mauve caractéristique de vraies solutions de fullerènes C₆₀ et ne permettaient pas d'obtenir l'effet conditionneur recherché.

Les fullerènes, contrairement aux deux autres formes du carbone, sont solubles dans un grand nombre de solvants organiques et confèrent à la solution une couleur caractéristique, par exemple mauve pour la molécule C₆₀ et rouge pour la molécule C₇₀.

On considère dans la présente invention que les fullerènes sont "à l'état dissous" ou "en solution" lorsque, à une concentration égale à 0,01 % en poids, dans un solvant organique donné à 20 °C, l'absorbance au maximum du pic d'absorption, situé dans un intervalle de longueurs d'ondes compris entre 300 et 400 nm, est supérieure ou égale à 0,3.

Il va de soi que les solvants organiques utilisés dans la présente invention pour la dissolution des fullerènes en vue d'une application sur les cheveux sont essentiellement des solvants organiques cosmétiquement acceptables, c'est-à-dire des composés liquides à température ambiante (20 °C) présentant une innocuité compatible avec une application topique, en particulier sur la peau, les lèvres et les phanères tels que les cheveux, cils, sourcils et ongles. De tels solvants cosmétiquement acceptables sont énumérés par exemple dans le *International Cosmetic Ingredient Dictionary and Handbook,* huitième édition, publié par The Cosmetic, Toiletry, and Fragrance Association (CTFA), Washington.

La demanderesse, après de nombreux essais, a constaté que des solvants organiques comportant au moins un noyau aromatique sont particulièrement appropriés pour la solubilisation des fullerènes.

Dans un mode de réalisation préféré de l'invention, le ou les solvants sont des esters d'acides mono- ou dicarboxyliques comportant au moins un noyau aromatique.

On peut citer à titre d'exemples de tels esters d'acides mono- ou dicarboxyliques aromatiques particulièrement préférés
- les benzoates d'alkyle en C₆₋₃₀ avec une chaîne alkyle linéaire ou ramifiée, de préférence les benzoates d'alkyle en C₈₋₁₈, et en particulier en C_{12-15,} et les dérivés oxypropylénés de ceux-ci. On peut citer à titre d'exemple de benzoates d'alkyle particulièrement préférés, le benzoate d'isostéaryle, le benzoate de stéaryle, le benzoate de stéaryle oxypropyléné, le benzoate de 2-octyldodécyle, le benzoate de béhényle et le benzoate de 2-éthylhexyle. Un mélange de benzoates d'alcools gras en C₁₂ à C₁₅ est commercialisé sous la dénomination FINSOLV® TN par la société WITCO ou sous la dénomination TEGOSOFT® TN par la société Goldschmidt.
- les phtalates de dialkyle en C₄₋₃₀, chaque chaîne alkyle pouvant être linéaire ou ramifiée, tels que les phtalates de dibutyle. Le terme "phtalate" englobe non seulement les esters dérivés de l'acide phtalique proprement dit (dérivé ortho) mais également ceux dérivés de l'acide téréphtalique (dérivé para) et de l'acide isophtalique (dérivé méta).

La concentration des fullerènes dans les compositions de la présente invention dépend d'un certain nombre de facteurs tels que le type de fullerène utilisé, la solubilité de celui-ci dans le solvant utilisé ou encore du mode d'application (application avec ou sans rinçage).

La demanderesse a obtenu des résultats tout à fait satisfaisants avec des concentrations, rapportées au poids total de la composition, comprises entre 0,0001 % et 3 %, de préférence entre 0,001 et 1 %, et en particulier entre 0,005 % et 0,1 %.

Les compositions de la présente invention peuvent contenir en outre un ou plusieurs solvants additionnels, différents des esters d'acides mono- ou dicarboxyliques comportant au moins un noyau aromatique décrits ci-dessus.

Ces solvants peuvent être volatils ou non et englobent notamment l'eau, les alcools aliphatiques ou aromatiques en C₂₋₁₈, les polyols en C₂₋₃₀, les alcanes en C₄₋₁₈, les silicones fluides volatiles ou non-volatiles, linéaires ou cycliques, l'acétone, la méthyléthylcétone, l'acétate de méthyle, l'acétate de butyle, l'acétate d'éthyle, le diméthoxyéthane ou le diéthoxyéthane.

Les solvants additionnels qui se sont avérés particulièrement avantageux pour une utilisation dans les compositions cosmétiques de la présente invention sont les silicones volatiles, en particulier les silicones cycliques volatiles, telles que le décaméthylpentasiloxane (D₅).

Les compositions de la présente invention peuvent contenir en outre un ou plusieurs principes actifs cosmétiques et/ou un ou plusieurs adjuvants de formulation. On peut citer à titre de tels principes actifs et adjuvants les huiles minérales, végétales ou animales, les cires oxyéthylénées ou non, les paraffines, les acides gras, les amides gras, les esters gras différents des solvants de l'invention, les alcools gras, les agents réducteurs, les agents oxydants, les séquestrants, les agents épaississants, les agents adoucissants, les agents anti-mousse, les agents hydratants, les agents émollients, les agents d'ajustement du pH, les agents plastifiants, les filtres solaires, les colorants directs, les précurseurs de colorants d'oxydation (bases et coupleurs), les pigments, les charges minérales, les argiles, les nacres, les parfums, les agents peptisants, les conservateurs, les agents tensioactifs anioniques, cationiques, non-ioniques, amphotères et zwitterionique, les polymères fixants, les polymères conditionneurs, les protéines, les vitamines.

L'homme du métier veillera à choisir le ou les solvants additionnels, le ou les principes actifs et/ou le ou les adjuvants de formulation de manière à ne pas nuire aux propriétés avantageuses intrinsèques des compositions de la présente invention. Il convient en particulier de s'assurer que la présence de ces ingrédients ne réduise pas notablement la solubilité des fullerènes dans la composition cosmétique finale.

Les compositions de la présente invention peuvent se présenter sous n'importe quelle forme permettant une application aisée ainsi qu'une répartition régulière sur la chevelure. Il peut s'agir par exemple d'une lotion, d'une mousse, d'un gel ou d'une émulsion.

Dans un mode de réalisation préféré de la présente invention, les compositions cosmétiques de la présente invention sont des shampooings ou des après-shampooings.

On peut envisager le conditionnement de ces compositions dans un dispositif permettant l'application par pulvérisation tel qu'un flacon-pompe ou un dispositif aérosol.

L'invention a donc également pour objet un dispositif aérosol contenant une composition cosmétique telle que décrite ci-dessus en présence d'au moins un agent propulseur. Cet agent propulseur est de préférence choisi parmi l'air, le gaz carbonique, l'azote, le diméthyléther, les hydrocarbures halogénés et les mélanges de ceux-ci.

L'invention est illustrée ci-après à l'aide d'exemples de réalisation et d'exemples comparatifs mettant en évidence l'importance de la solubilisation des fullerènes pour l'obtention de l'effet de conditionnement recherché.

### Exemple 1

### Préparation d'une solution de fullerènes dans un mélange de benzoates d'alkyle en C₁₂₋₁₅

On introduit 10 mg de fullerènes C₆₀ (purs à 99,9 %) commercialisés par la société Mer Corporation sous la référence MR6HP dans 100 g d'un mélange de benzoates d'alkyle en C₁₂₋₁₅ commercialisé sous la dénomination FINSOLV® TN par la société WITCO. On expose la suspension ainsi préparée pendant 30 minutes à des ultrasons (Cuve à ultrasons Branson 2210) de manière à obtenir une solution de couleur mauve.

Dans le spectre UV-visible obtenu avec un spectrophotomètre CARY 100 SCAN (Variant), on observe un pic d'absorption avec un maximum d'absorption à 331 nm. L'absorbance au maximum d'absorption est d'environ 0,8.

A titre de comparaison, on répète le protocole ci-dessus en remplaçant le FINSOLV® TN par de l'adipate de diisobutyle (commercialisé par la société Stéarinerie Dubois Fils). L'adipate de diisobutyle est un des solvants organiques cosmétiquement acceptables utilisés, dans la demande JP 9278625, en tant que composant huileux pour la "solubilisation" des fullerènes.

Dans le spectre UV-visible de ce mélange, tracé dans les mêmes conditions que précédemment il apparaît clairement que l'adipate de diisobutyle est incapable de dissoudre les fullerènes C₆₀ à une concentration de 0,01 % en poids.

### Exemple 2

### Effet cosmétique de solutions de fullerènes C₆₀ en application non rincée

On prépare les compositions A (selon l'invention) et B (comparative) suivantes en mettant en suspension 1 g de fullerènes C₆₀ respectivement dans 1 litre de benzoate d'alkyle en C₁₂₋₁₅ et 1 litre d'adipate de diisobutyle. On expose ces mélanges pendant 30 minutes à 20 °C à des ultrasons dans une cuve à ultrasons (Branson 2210), puis on ajoute à chacun des mélanges 1 litre de décaméthylpentasiloxane (D₅).

**Tableau 1**

| | **Composition A** (selon l'invention) | **Composition B** (comparative) |
|---|---|---|
| benzoate d'alkyle en C₁₂₋₁₅^{[1]} | 49,98 % | - |
| adipate de diisobutyle^{[2]} | - | 49,98 % |
| cyclopentasiloxane^{[3]} | 49,98 % | 49,98 % |
| fullerènes C₆₀^{[4]} | 0,05 % | 0,05 % |
| couleur de la composition | mauve (solution) | brune (suspension) |

| | | |
|---|---|---|
| ^{[1]} Finsolv® TN, Witco | | |
| ^{[2]} commercialisé par la société Stéarinerie Dubois Fils | | |
| ^{[3]} DC 2-5252 C, Dow Corning | | |
| ^{[4]} purs à 99,9 %, commercialisés sous la référénce MR6HP par Mer Corp. | | |

On prépare à partir des compositions A et B du tableau 1 ci-dessus les après-shampooings I (selon l'invention) et II (comparatif) ci-après, ainsi que des après-shampooings comparatifs III et IV ne contenant pas de fullerènes.

**Tableau 2**

| | Après-shampooing I (selon l'invention) | Après-shampooing II (comparatif) | Après-shampooing III (comparatif) | Après-shampooing IV (comparatif) |
|---|---|---|---|---|
| Composition A | 10 % | - | - | - |
| Composition B | - | 10 % | - | - |
| Pentadiméthylsiloxane | - | - | 5 % | 5 % |
| Adipate de dibutyle | - | - | - | 5 % |
| Benzoate d'alkyle en C₁₂₋₁₅ | - | - | 5 % | - |
| Cyclopentasiloxane Diméthicone copolyol | 0,5 % | 0,5 % | 0,5 % | 0,5 % |
| Propylèneglycol | 2,5 % | 2,5 % | 2,5 % | 2,5 % |
| Conservateur | qs | qs | qs | qs |
| parfum | qs | qs | qs | qs |
| eau | qsp 100 % | qsp 100 % | qsp 100 % | qsp 100 % |

On applique chacun des après-shampooing I, II, III et IV sur 10 mèches de cheveux caucasiens naturels châtains d'une longueur de 20 cm, à raison de 1 g par mèche.

Après un temps de pose de 10 minutes, on sèche les cheveux sans les avoir rincés auparavant.

Un panel de 10 experts indique systématiquement que l'après-shampooing I selon l'invention contenant des fullerènes C₆₀ à l'état dissous, confère aux cheveux un aspect plus brillants que les après-shampooings comparatifs contenant des fullerènes en suspension ou exempts de fullerènes. Les cheveux ainsi traités ne présentent pas de toucher gras ou chargé.

La composition comparative II contenant des fullerènes C₆₀ à l'état agrégé, non-dissous, possède des propriétés cosmétiques très proches des compositions comparatifs III et IV exemptes de fullerènes.

Cet exemple montre que l'application de fullerènes sous forme non-dissoute (suspension de couleur brune) n'apporte pas d'avantages cosmétiques particuliers.

### Exemple 3

### Effet cosmétique des solutions de fullerènes C₆₀ en application rincée

On prépare de manière analogue à l'exemple 2 l'après-shampooing (i) selon l'invention et les après-shampooings (ii) à (iv) comparatifs ci-après.

**Tableau 3**

| | Après-shampooing (i) (selon l'invention) | Après-shampooing (ii) (comparatif) | Après-shampooing (iii) (comparatif) | Après-shampooing (iv) (comparatif) |
|---|---|---|---|---|
| Composition A | 20 % | - | | - |
| Composition B | - | 20 % | | - |
| Pentadiméthylsiloxane | | | 10% | 10 % |
| Adipate de dibutyle | | | - | 10 % |
| Benzoate d'alkyle en C₁₂₋₁₅ | | | 10 % | - |
| Diméthicone copolyol | 0,5 % | 0,5 % | 0,5 % | 0,5 % |
| Propylèneglycol | 2,5 % | 2,5 % | 2,5 % | 2,5 % |
| Behentrimonium chloride | 1,2 % | 1,2 % | 1,2 % | 1,2 % |
| eau | qsp 100 % | qsp 100 % | qsp 100 % | qsp 100 % |

On applique chacun des après-shampooings du tableau 3 sur 10 mèches de cheveux caucasiens naturels châtains d'une longueur de 20 cm, à raison de 1 g par mèche. Après un temps de pose de deux minutes, on rince les mèches à l'eau, puis on les sèche au casque pendant 30 minutes à 70 °C.

Un panel de 10 experts indique systématiquement que l'après-shampooing (i) selon l'invention contenant des fullerènes C₆₀ à l'état dissous (composition de coloration mauve), confère aux cheveux un aspect plus brillant que les après-shampooings comparatifs contenant des fullerènes en suspension (composition de coloration brune) ou exempts de fullerènes. Les cheveux ainsi traités ne présentent pas le toucher gras ou chargé obtenu classiquement avec des silicones.

La composition comparative (ii) contenant des fullerènes C₆₀ à l'état agrégé, non-dissous, possède des propriétés cosmétiques très proches des compositions comparatifs (iii) et (iv) exemptes de fullerènes. La présence de fullerènes C₆₀ sous fonne agrégée, non-dissoute dans la composition capillaire n'apporte donc pas d'avantages cosmétiques particuliers.

## Revendications

1. Utilisation de fullerènes, non-modifiés par greffage de fonctions chimiques sur le squelette carboné, à l'état dissous dans un ou plusieurs solvants organiques comportant au moins un noyau aromatique, pour le conditionnement et/ou l'augmentation de la brillance des cheveux.

2. Utilisation selon la revendication 1, **caractérisée par le fait que** les fullerènes sont des fullerènes C₆₀.

3. Utilisation selon la revendication 1 ou 2, **caractérisée par le fait que** le ou les solvants organiques sont choisis parmi les esters d'acides mono- ou dicarboxyliques comportant au moins un noyau aromatique.

4. Utilisation selon la revendication 3, **caractérisée par le fait que** le ou les solvants organiques sont choisis parmi les benzoates d'alkyle en C₆₋₃₀ et les phtalates de dialkyle en C₄₋₃₀.

5. Utilisation selon la revendication 4, **caractérisée par le fait que** le ou les solvants organiques sont choisis parmi les benzoates d'alkyle en C₈₋₁₈, de préférence parmi les benzoates d'alkyle en C₁₂₋₁₅.

6. Procédé de traitement des cheveux, comprenant l'application et l'étalement sur les cheveux, d'une solution de fullerènes non-modifiés par greffage de fonctions chimiques sur le squelette carboné, dans un ou plusieurs solvants organiques comportant au moins un noyau aromatique, éventuellement le rinçage des cheveux ainsi traités avec de l'eau, puis le séchage des cheveux

7. Composition cosmétique, destinée au traitement des cheveux, contenant des fullerènes non-modifiés par greffage de fonctions chimiques sur le squelette carboné, à l'état dissous dans un ou plusieurs solvants organiques choisis parmi les esters d'acides mono- ou dicarboxyliques comportant au moins un noyau aromatique.

8. Composition selon la revendication 7, **caractérisée par le fait que** les fullerènes sont des fullerènes C₆₀.

9. Composition selon la revendication 7 ou 8, **caractérisée par le fait que** le ou les solvants organiques cosmétiquement acceptables sont choisis parmi les benzoates d'alkyle en C₆₋₃₀ et les phtalates de dialkyle en C₄₋₃₀.

10. Composition selon la revendication 9, **caractérisée par le fait que** le ou les solvants organiques sont choisis parmi les benzoates d'alkyle en C₈₋₁₈, de préférence parmi les benzoates d'alkyle en C₁₂₋₁₅.

11. Composition selon l'une quelconque des revendications 7 à 10, **caractérisée par le fait que** la concentration des fullerènes est comprise entre 0,0001 % et 3 %, de préférence entre 0,001 et 1 %, et en particulier entre 0,005 % et 0,1 %, rapporté au poids total de la composition.

12. Composition selon l'une quelconque des revendications 7 à 11, **caractérisée par le fait qu'**elle contient en outre un ou plusieurs solvants différents des esters d'acides mono- ou dicarboxyliques comportant au moins un noyau aromatique, choisis parmi l'eau, les alcools aliphatiques ou aromatiques en C₂₋₁₈, les polyols en C₂₋₃₀, les alcanes en C₄₋₁₈, les silicones fluides volatiles ou non-volatiles, linéaires ou cycliques, l'acétone, la méthyléthylcétone, l'acétate de méthyle, l'acétate de butyle, l'acétate d'éthyle, le diméthoxyéthane ou le diéthoxyéthane.

13. Composition selon la revendication 12, **caractérisée par le fait que** le solvant est une silicone cyclique volatile, de préférence le décaméthylpentasiloxane (D₅).

14. Composition selon l'une quelconque des revendications 7 à 13, **caractérisée par le fait qu'**elle contient en outre un ou plusieurs principes actifs cosmétiques et/ou un ou plusieurs adjuvants de formulation.

15. Composition selon l'une quelconque des revendications précédentes, **caractérisée par le fait qu'**elle se présente sous forme de lotion, de mousse, de gel ou d'émulsion.

16. Composition selon l'une quelconque des revendications précédentes, **caractérisée par le fait qu'**il s'agit d'un shampooing ou d'un après-shampooing.

17. Dispositif aérosol contenant une composition cosmétique selon l'une quelconque des revendications 7 à 16 en présence d'au moins un agent propulseur.

18. Dispositif aérosol selon la revendication 17, **caractérisée par le fait que** l'agent propulseur est choisi parmi l'air, le gaz carbonique, l'azote, le diméthyléther, les hydrocarbures halogénés et les mélanges de ceux-ci.

## Claims

1. Use of fullerenes not modified by grafting of chemical functions onto the carbon backbone, dissolved in one or more organic solvents comprising at least one aromatic nucleus, for conditioning and/or increasing the sheen of the hair.

2. Use according to Claim 1, **characterized in that** the fullerenes are C₆₀ fullerenes.

3. Use according to Claim 1 or 2, **characterized in that** the organic solvents are chosen from monocarboxylic or dicarboxylic acid esters comprising at least one aromatic nucleus.

4. Use according to Claim 3, **characterized in that** the organic solvent(s) is (are) chosen from C₆₋₃₀ alkyl benzoates and C₄₋₃₀ dialkyl phthalates.

5. Use according to Claim 4, **characterized in that** the organic solvent(s) is (are) chosen from C₈₋₁₈ alkyl benzoates and preferably from C₁₂₋₁₅ alkyl benzoates.

6. Hair treatment process comprising the application and spreading on the hair of a solution of fullerenes not modified by grafting of chemical functions onto the carbon backbone, in one or more organic solvents comprising at least one aromatic nucleus, optionally the rinsing of the hair thus treated with water, followed by drying of the hair.

7. Cosmetic composition for treating the hair, containing fullerenes not modified by grafting of chemical functions onto the carbon backbone, dissolved in one or more organic solvents chosen from monocarboxylic or dicarboxylic acid esters comprising at least one aromatic nucleus.

8. Composition according to Claim 7, **characterized in that** the fullerenes are C₆₀ fullerenes.

9. Composition according to Claim 7 or 8, **characterized in that** the cosmetically acceptable organic solvent (s) is (are) chosen from C₆₋₃₀ alkyl benzoates and C₄₋₃₀ dialkyl phthalates.

10. Composition according to Claim 9, **characterized in that** the organic solvent(s) is (are) chosen from C₈₋₁₈ alkyl benzoates and preferably from C₁₂₋₁₅ alkyl benzoates.

11. Composition according to any one of Claims 7 to 10, **characterized in that** the fullerene concentration is between 0.0001% and 3%, preferably between 0.001% and 1% and in particular between 0.005% and 0.1% relative to the total weight of the composition.

12. Composition according to any one of Claims 7 to 11, **characterized in that** it also contains one or more solvents other than monocarboxylic or dicarboxylic acid esters comprising at least one aromatic nucleus, chosen from water, aliphatic or aromatic C₂₋₁₈ alcohols, C₂₋₃₀ polyols, C₄₋₁₈ alkanes, volatile or non-volatile, linear or cyclic fluid silicones, acetone, methyl ethyl ketone, methyl acetate, butyl acetate, ethyl acetate, dimethoxyethane and diethoxyethane.

13. Composition according to Claim 12, **characterized in that** the solvent is a volatile cyclic silicone, preferably decamethylpentasiloxane (D₅).

14. Composition according to any one of Claims 7 to 13, **characterized in that** it also contains one or more cosmetic active principles and/or one or more formulation adjuvants.

15. Composition according to any one of the preceding claims, **characterized in that** it is in the form of a lotion, a mousse, a gel or an emulsion.

16. Composition according to any one of the preceding claims, **characterized in that** it is a shampoo or a hair conditioner.

17. Aerosol device containing a cosmetic composition according to any one of Claims 7 to 16 in the presence of at least one propellant.

18. Aerosol device according to Claim 17, **characterized in that** the propellant is chosen from air, carbon dioxide, nitrogen, dimethyl ether and halogenated hydrocarbons, and mixtures thereof.

## Patentansprüche

1. Verwendung von Fullerenen, die nicht durch das Aufpfropfen von chemischen Gruppen auf das Kohlenstoffgerüst modifiziert sind, die in einem oder mehreren organischen Lösemitteln gelöst sind, die mindestens einen aromatischen Ring aufweisen, für die Konditionierung der Haare und/oder die Verbesserung des Glanzes der Haare.

2. Verwendung nach Anspruch 1, **dadurch gekennzeichnet, dass** es sich bei den Fullerenen um C₆₀-Fullerene handelt.

3. Verwendung nach Anspruch 1 oder 2, **dadurch gekennzeichnet, dass** das oder die organischen Lösemittel unter den Estern von Mono- oder Dicarbonsäuren ausgewählt werden, die mindestens einen aromatischen Ring aufweisen.

4. Verwendung nach Anspruch 3, **dadurch gekennzeichnet, dass** das oder die organischen Lösemittel unter den C₆₋₃₀-Alkylbenzoaten und den C₄₋₃₀-Dialkylphthalaten ausgewählt werden.

5. Verwendung nach Anspruch 4, **dadurch gekennzeichnet, dass** das oder die organischen Lösemittel unter den C₈₋₁₈-Alkylbenzoaten, vorzugsweise unter den C₁₂₋₁₅-Alkylbenzoaten, ausgewählt werden.

6. Verfahren zur Behandlung der Haare, das das Auftragen und Verteilen einer Lösung von Fullerenen, die nicht durch das Auf pfropfen von chemischen Gruppen auf das Kohlenstoffgerüst modifiziert sind, in einem oder mehreren organischen Lösemitteln, die mindestens einen aromatischen Ring aufweisen, auf den Haaren und gegebenenfalls das Spülen der so behandelten Haare mit Wasser und dann das Trocknen der Haare umfasst.

7. Kosmetische Zusammensetzung, die für die Behandlung der Haare vorgesehen ist, die Fullerene enthält, die nicht durch das Aufpfropfen von chemischen Gruppen auf das Kohlenstoffgerüst modifiziert sind, die in einem oder mehreren organischen Lösemitteln gelöst sind, die unter den Estern von Mono- oder Dicarbonsäuren ausgewählt werden, die mindestens einen aromatischen Ring enthalten. ,

8. Zusammensetzung nach Anspruch 7, **dadurch gekennzeichnet, dass** es sich bei den Fullerenen um C₆₀-Fullerene handelt.

9. Zusammensetzung nach Anspruch 7 oder 8, **dadurch gekennzeichnet, dass** das oder die kosmetisch akzeptablen organischen Lösemittel unter den C₆₋₃₀-Alkylbenzoaten und den C₄₋₃₀-Dialkylphthalaten ausgewählt werden.

10. Zusammensetzung nach Anspruch 9, **dadurch gekennzeichnet, dass** das oder die organischen Lösemittel unter den C₈₋₁₈-Alkylbenzoaten, vorzugsweise unter den C₁₂₋₁₅-Alkylbenzoaten, ausgewählt werden.

11. Zusammensetzung nach einem der Ansprüche 7 bis 10, **dadurch gekennzeichnet, dass** die Konzentration der Fullerene im Bereich von 0,0001 bis 3 %, vorzugsweise 0,001 bis 1 % und vor allem 0,005 bis 0,1 %, bezogen auf das Gesamtgewicht der Zusammensetzung, liegt.

12. Zusammensetzung nach einem der Ansprüche 7 bis 11, **dadurch gekennzeichnet, dass** sie außerdem ein oder mehrere Lösemittel enthält, die verschieden von den Estern von Mono- oder Dicarbonsäuren sind, die mindestens einen aromatischen Ring enthalten, die unter Wasser, den aliphatischen oder aromatischen C₂₋₁₈-Alkoholen, den C₂₋₃₀-Polyolen, den C₄₋₁₈-Alkanen, den flüchtigen oder nichtflüchtigen, geradkettigen oder zyklischen fluiden Siliconen, Aceton, Methylethylketon, Methylacetat, Butylacetat, Ethylacetat, Dimethoxyethan und Diethoxyethan ausgewählt werden.

13. Zusammensetzung nach Anspruch 12, **dadurch gekennzeichnet, dass** es sich bei dem Lösemittel um ein flüchtiges zyklisches Silicon, vorzugsweise Decamethylpentasiloxan (D₅), handelt.

14. Zusammensetzung nach einem der Ansprüche 7 bis 13, **dadurch gekennzeichnet, dass** sie außerdem einen oder mehrere kosmetische Wirkstoffe und/oder einen oder mehrere Formulierungszusatzstoffe enthält.

15. Zusammensetzung nach einem der vorhergehenden Ansprüche, **dadurch gekennzeichnet, dass** sie in Form einer Lotion, eines Schaums, eines Gels oder einer Emulsion vorliegt.

16. Zusammensetzung nach einem der vorhergehenden Ansprüche, **dadurch gekennzeichnet, dass** es sich um ein Haarwaschmittel oder einen Konditioner handelt.

17. Aerosolvorrichtung, die eine kosmetische Zusammensetzung nach einem der Ansprüche 7 bis 16 in Gegenwart von mindestens einem Treibmittel enthält.

18. Aerosolvorrichtung nach Anspruch 17, **dadurch gekennzeichnet, dass** das Treibmittel unter Luft, Kohlendioxid, Stickstoff, Dimethylether, halogenierten Kohlenwasserstoffen und den Gemischen dieser Stoffe ausgewählt wird.
